# EUROPÄISCHE PATENTSCHRIFT

(11) **EP 3 177 236 B1**
(45) Veröffentlichungstag und Bekanntmachung des Hinweises auf die Patenterteilung: **16.12.2020**
(21) Anmeldenummer: 15747788.6
(22) Anmeldetag: 03.08.2015
(51) Int. Cl.: A61F 2/78, A61F 2/50, A61F 2/74, A61F 2/80

(54) **LINER**
LINER
MANCHON

(30) Priorität: 05.08.2014 DE 102014011374
(43) Veröffentlichungstag der Anmeldung: 14.06.2017
(73) Patentinhaber: Ottobock SE & Co. KGaA, 37115 Duderstadt (DE)
(72) Erfinder: ANHALT, Klaus Peter, 37434 Rhumspringe (DE); KIELE, Susann, 37115 Duderstadt (DE)
(74) Vertreter: Gramm, Lins & Partner Patent- und Rechtsanwälte PartGmbB
(86) Internationale Anmeldenummer: PCT/EP2015/067800
(87) Internationale Veröffentlichungsnummer: WO 2016/020317

(56) Entgegenhaltungen:
- EP-A1- 2 353 550
- EP-A2- 0 346 697
- EP-A2- 0 363 654
- WO-A2-2009/017762
- US-A1- 2010 274 364

## Beschreibung

Die Erfindung betrifft einen Liner zur Anlage an einer Hautoberfläche mit einem Grundkörper aus einem flexiblen, wasserundurchlässigen Material mit einer im angelegten Zustand von der Hautoberfläche abgewandten Außenseite und einer im angelegten Zustand der Hautoberfläche zugewandten Innenseite.

Liner aus dem Stand der Technik sind dafür vorgesehen, an einen Stumpf einer Gliedmaße angelegt zu werden, um eine Schnittstelle insbesondere zu einer Prothese herzustellen. Die Liner können als Kunststoff- oder Silikonliner ausgebildet sein und weisen in der Regel einen geschlossenen Querschnitt auf, so dass sie den Stumpf luftdicht umschließen. Die Innenseite kann mit einer auf der Hautoberfläche haftenden Beschichtung versehen sein. Der Stumpf wird zusammen mit dem Liner in einen Prothesenschaft eingeführt und legt den Liner in dem Prothesenschaft über eine Verriegelungseinrichtung oder über einen Unterdruck fest. Die Erzeugung des Unterdruckes im Rahmen der sogenannten Saugschaftlinertechnologie erfolgt dadurch, dass der Prothesenschaft gegenüber dem Prothesenliner luftdicht abgedichtet und aus dem Zwischenraum zwischen dem Prothesenliner und dem Prothesenschaft die darin vorhandene Luft abgesaugt oder herausgedrückt wird. Ein Rückströmen in den Zwischenraum wird über ein Rückschlagventil verhindert.

Die WO 2012/051385 A1 betrifft einen Prothesenliner, der als Interface zwischen einem Stumpf und einem Prothesenschaft ausgebildet ist. Der Prothesenliner weist einen langgestreckten, im Wesentlichen konischen Grundkörper auf, in dem ein Volumensteuerkissen angeordnet ist. An der Außenseite des Prothesenliners sind im Distalbereich radial nach außen gerichtete, umlaufende Dichtlippen angeordnet, um eine verbesserte Abdichtung gegenüber der inneren Oberfläche eines Prothesenschaftes bereitzustellen.

Eine ähnliche Ausgestaltung eines Liners ist in der US 8,052,760 B2 beschrieben, bei der im äußeren Umfang des Prothesenliners Dichtelemente angeordnet sind, um eine verbesserte Abdichtung des Zwischenraumes zwischen dem Prothesenliner und dem Prothesenschaft zu gewährleisten.

Die DE 101 53 796 A1 betrifft unter anderem einen Liner zur Verwendung mit einem becherförmigen Prothesenschaft, der an seinem distalen Ende eine Öffnung aufweist, durch die eine Schnur zum Einziehen des Liners hindurchgeführt werden kann. Über einen mechanischen Verriegelungsstift wird der Liner in dem Prothesenschaft verriegelt.

Das Problem bei Prothesenlinern dieser Art besteht darin, dass die natürlichen Wärmeregulationsmechanismen wie Wärmeleitung, Wärmeströmung, Wärmestrahlung und Verdunstung des Menschen bei einem vollflächigen unmittelbaren Hautkontakt mit der Lineroberfläche nur eingeschränkt wirksam sind. Ein Wärmestrom in Form von Konvektion kann bei einem vollständigen Kontakt zur Haut nicht stattfinden, auch ist die Verdunstung von Schweiß durch die verwendeten Materialien nur eingeschränkt möglich.

Die US 8,182,547 B2 sieht daher auf der Innenseite eines Liners eine viellagige Textilschicht vor, wobei der proximale Rand eine luftdichte Abdichtung gewährleistet. Über Belüftungskanäle kann Luft in den Zwischenraum zwischen der Textilschicht und der Hautoberfläche eingeleitet und abgesaugt werden, um sowohl den Unterdruck als auch die Belüftung zu steuern.

Die US 6,974,484 B2 sieht zum Abtransport von Schweiß das Einlegen einer osmotischen Membran vor, die von einem Liner abgedeckt wird. Die osmotische Membran ermöglicht den Abtransport von Feuchtigkeit von dem Stumpf, sperrt aber gegen einen Rückstrom.

Die US 2012/0191218 A1 betrifft ein vakuumunterstütztes Trägersystem für Protheseneinrichtungen an unteren Extremitäten mit einem luftundurchlässigen Schaft mit Anschlüssen für eine Vakuumquelle, einem Liner, der an seinen Rändern luftdicht abgedichtet ist und zwischen seinen Rändern poröse Bereiche aufweist. Der Liner ist gegenüber dem Schaft abgedichtet und zwischen dem Liner und dem Schaft ist eine luftdurchlässige Verteilschicht z.B. aus einem Textil angeordnet, um das an den Schaft angelegte Vakuum zu verteilen. Über eine Pumpe können Luft und Feuchtigkeit von der Hautoberfläche weg transportiert und aus dem Schaft abgesaugt werden.

Die WO 2009/017762 A2 betrifft einen Liner zur Verwendung in prothetischen oder orthetischen Vorrichtungen. Der Liner weist eine innere Schicht mit rutschhemmenden Komponenten auf, die zumindest einen Teil des Umfanges der inneren Lineroberfläche bilden. Die innere Schicht bildet eine Vielzahl von Öffnungen aus, die zu einem porösen Element führen. Durch die Öffnungen in der inneren Schicht wird die Feuchtigkeit von dem Stumpf weg und durch das poröse Element an die Umgebung abgegeben. Die Öffnungen können bereichsweise auf der Innenseite angeordnet sein. In dem porösen Material können absorbierende Elemente gespeichert sein. Eine geschlossene Außenschicht kann die perforierte Schicht umgeben. Eine Variante sieht vor, dass der Liner aus zwei oder mehreren Schichten zusammenhängender Polymerbälle besteht, wobei die aneinander anliegenden Bälle miteinander verbunden sind und den gleichen Durchmesser haben können.

Die US 8,282,686 B2 betrifft eine Prothesenschaftanordnung zur Aufnahme eines Amputationsstumpfes mit einer formstabilen Außenschale, die der Form des aufzunehmenden Stumpfes entspricht. Eine luftdurchlässige Innenschicht ist auf der Innenseite der Außenschale an ausgewählten Bereichen angeordnet, um mit der Hautoberfläche in Kontakt zu treten und das angelegte Vakuum zu verteilen. Die Innenschicht ist aus einem mehrlagigen Textil ausgebildet. An der Außenschale ist ein Einlass und ein mit einer Vakuumquelle verbundener Auslass vorgesehen.

Die EP 2 353 550 A1 beschreibt ein Bauteil für eine künstliche Gliedmaße mit einem Grundkörper, der aus einem luftdurchlässigen Gewebe hergestellt ist. Eine Vielzahl von Vorsprüngen aus Silikon sind auf der Innenseite und der Außenseite des Grundkörpers angeordnet, wobei benachbarte Vorsprünge voneinander beabstandet sind.

Die US 2010/274364 A1 betrifft eine einstellbare Prothese mit einem darin angeordneten Liner, der eine Ventilationsschicht sowie eine Membran aufweist. Die Ventilationsschicht weist radial nach außen gerichtete Durchgangslöcher auf, die Feuchtigkeit zu der außen liegenden Membran transportieren. In einer Variante ist vorgesehen, dass in Längserstreckung des Liners gerichtete Kanäle auf der Innenseite mit einer feuchtigkeitsdurchlässigen Membran abgedeckt sind. In einer weiteren Variante sind längsgerichtete Kanäle in einer Belüftungsschicht oder in einem Liner ausgebildet, die über Durchgangslöcher mit der Oberfläche des Stumpfes in Kontakt stehen.

Die EP 0 363 654 A2 betrifft eine Prothese mit Mitteln zum Belüften einer Grenzoberfläche zwischen einem Stumpf und einer Prothese. Die Belüftungsmittel sehen eine Aufnahme vor, die luftdurchlässig ist und eine Form aufweist, die dem Stumpf entspricht. Eine Lüftungseinrichtung kann über einen Verbindungskanal mit der Aufnahme verbunden sein. Die Aufnahme kann als Hülse ausgebildet sein, die auch aus einem porösen Material hergestellt sein kann, das ein Durchströmen des Lüftungsmediums ermöglicht. Auf der Innenseite der Hülse können Kanäle und/oder Öffnungen eingearbeitet sein.

Die EP 0346697 A2 betrifft eine orthopädische Vorrichtung wie eine Prothese, Gipsschiene oder Luftschiene, die eine auf der Haut des Patienten aufliegende Aufstandsfläche aufweist. Die Aufstandsfläche ist mit einem flächigen, flexiblen Ventilationsschichtmaterial ausgekleidet. In dem Material sind Längsluftkanäle und Querluftkanäle vorhanden, wobei das Material eine Druckstabilität aufweist, die bei einem normalen Gebrauch die Luftleitfähigkeit in Längsrichtung und Querrichtung aufrechterhält. Das Material kann verschieden strukturiert sein, beispielsweise offenporig, rippenartig, genoppt, mit Stegen versehen, netzartig dreidimensional oder dergleichen, wobei wesentlich ist, dass Längsluftkanäle und mit diesen in Verbindung stehende Querluftkanäle bis zu mindestens einer Oberflächliche hin entstehen.

Aufgabe der vorliegenden Erfindung ist es, einen Liner bereitzustellen, mit dem einerseits eine gute Wärmeregulierung und andererseits ein angenehmes Tragempfinden erreicht werden kann.

Erfindungsgemäß wird diese Aufgabe durch einen Liner mit den Merkmalen des Hauptanspruches gelöst. Vorteilhafte Ausgestaltungen und Weiterbildungen der Erfindung sind in den Unteransprüchen, der Beschreibung und den Figuren aufgeführt.

Der Liner zur Anlagen an einer Hautoberfläche mit einem Grundkörper aus einem flexiblen, wasserundurchlässigen Material mit einer im angelegten Zustand von der Hautoberfläche abgewandten Außenseite und einer im angelegten Zustand der Hautoberfläche zugewandten Innenseite sieht vor, dass auf der Innenseite zur Hautoberfläche hin offene Kanäle angeordnet sind. Dadurch ist es möglich, dass einerseits ein Kontakt der Innenseite des Liners mit der Hautoberfläche stattfindet und andererseits ein Wärmestrom in Form von Konvektion und einer Verdunstung von Schweiß gleichermaßen ermöglicht wird. Es wird somit ein unmittelbarer Kontakt des Liners sowohl zu der Hautoberfläche als auch zu der Innenseite des Prothesenschaftes hergestellt und darüber der Prothesenschaft mit der Gliedmaße gekoppelt. Darüber hinaus wird durch die und entlang der Kanäle zwischen den Anlageflächen ein unmittelbar an der Hautoberfläche entlang geführter Wärme- und Feuchtigkeitstransport durchgeführt, wodurch die natürlichen Wärmeregulationsmechanismen aufrecht erhalten bleiben und angenehmes Traggefühl vermitteln. Der Liner ist aus wasserundurchlässigen Materialien oder einem einzigen Material hergestellt, was verhindert, dass Feuchtigkeit von der der Hautoberfläche durch das Material hindurch abtransportiert wird, wobei Materialien so beschaffen sind, dass sie einen Wasserdurchtritt in flüssiger Form verhindern. Als wasserundurchlässige Materialien eignen sich insbesondere Elastomere, Silikone und gummiartige Werkstoffe oder auch geschlossenporige Schäume. Die Liner dienen als Interface zwischen dem Stumpf und einem Prothesenschaft. Durch die auf der Oberfläche der Haut stattfindende Verdunstung findet eine effektive Kühlung der Hautoberfläche statt, was den Tragekomfort merklich erhöht. Der Grundkörper weist vorteilhafterweise eine geschlossene Außenseite auf, um zu verhindern, dass Feuchtigkeit nach außen gelangt, so dass sich keine Feuchtigkeit zwischen dem Liner und dem Prothesenschaft ansammeln kann.

Der Grundkörper kann einen geschlossenen Querschnitt mit einer proximalen Einstiegsöffnung aufweisen, so dass er als im Wesentlichen konischer Hohlkörper ausgebildet ist. Das distale Ende des Prothesenliners ist üblicherweise geschlossen ausgebildet oder weist zumindest eine Öffnung zum Abtransport von Feuchtigkeit auf.

Alternativ dazu kann der Grundkörper eine Öffnung am distalen Ende aufweisen oder aber als offener Querschnitt mit Verschlusselementen versehen sein, so dass der zunächst flächige Prothesenliner um den Stumpf herum angelegt und über die Verschlusselemente unter Ausbildung eines ringförmigen Liners daran festgelegt werden kann.

Auf der Außenseite des Liners kann eine Textilschicht aufgebracht oder befestigt sein, die die Außenoberfläche des Liners ganz oder teilweise abdeckt. Die Textilschicht wird aufgrund der geschlossenen, wasserundurchlässigen Oberfläche des Liners von der innerhalb des Liners befindlichen Feuchtigkeit ferngehalten, der Liner wirkt als Feuchtigkeitssperre zwischen der Hautoberfläche und der Außenseite des Liners bzw. der Textilschicht. Die Verdunstung innerhalb des Liners in den Kanälen oder Zwischenräumen bewirkt eine Kühlung, ohne die Außenseite des Liners zu beeinträchtigen oder einen Stoffdurchtritt nach außen zu erlauben.

Auf der Außenseite des Grundkörpers kann eine Beschichtung aufgebracht sein, die vom Material des Grundkörpers abweicht. Dadurch ist es möglich, mehrschichtige Liner mit unterschiedlichen Eigenschaften herzustellen, so dass beispielsweise auf der Innenseite besonders hautverträgliche Materialien mit einer guten Haftfähigkeit eingesetzt werden können, während auf der Außenseite besonders stabile und abriebfeste Materialien Einsatz finden können. Die äußere Beschichtung kann auch ein Textil sein oder textile Komponenten aufweisen. Auf der Außenseite des Grundkörpers kann auch ein Bezug oder ein Überzug reversibel aufgebracht sein, so dass der Liner als Innenliner mit Kanälen oder Noppen und einem Außenliner gebildet sein kann.

Die zur Hautoberfläche hin offenen Kanäle sind vorteilhafterweise von Noppen ausgebildet, so dass die Kanäle um die Noppen herumgeführt sind. Dadurch ist es möglich, eine große Fläche bereitzustellen, entlang der die Luft über die Hautoberfläche strömen kann, um einen Wärme- und Feuchtigkeitstransport zu erreichen. Durch die Ausgestaltung der Kanäle durch die Noppen ist es möglich, sich kreuzenden und in verschiedene Richtungen orientierte Kanäle bereitzustellen, so dass neben der üblichen Strömungsrichtung, die der Konvektion folgt, auch quer oder schräg dazu verlaufende Strömungsrichtungen und Luftströmungen erfolgen können, die beispielsweise einer Pumpbewegung aufgrund der dynamischen Belastung beim der Benutzung einer Prothese folgt. Durch sich kreuzenden oder schneidende Kanäle wird die Entlüftung und der Wärme- und Feuchtigkeitstransport weiter verbessert. Alternativ oder ergänzend zu den Noppen können die Kanäle auch durch Stege ausgebildet sein, die auf der Innenseite des Grundkörpers angeordnet sind.

Die Noppen oder Stege sind einstückig mit dem Grundkörper ausgebildet, beispielsweise im Herstellverfahren des Liners. Zur Herstellung der Noppen oder Stege können in eine im Wesentlichen glattwandige Innenwand Kanäle eingebracht, beispielsweise eingefräst oder eingeschnitten werden, alternativ sind die Noppen oder Stege in der Form eingebracht und werden bei dem Herstellprozess des Liners, insbesondere beim Gießen, gebildet. Die Noppen oder Stege sind dann integraler Bestandteil des einstückig ausgebildeten Liners. Noppen sind dabei als Erhebungen anzusehen, die über eine Grundfläche herausstehen, wobei die einzelnen Erhebungen isoliert voneinander sind, wodurch sich eine strukturierte Oberfläche mit Erhebungen und Vertiefungen ausbildet. Die Erhebungen und Vertiefungen können regelmäßig oder unregelmäßig auf der Innenseite verteilt sein, ebenso ist es möglich, dass die Noppen voneinander verschiedene Formen und Größen aufweisen, um eine Anpassung an lokale Belastungen vornehmen zu können. Stege weisen im Gegensatz zu den Noppen eine größere Längserstreckung auf und können sich von einem Ende des Liners bis zum anderen Ende erstrecken.

Die Noppen und/oder Stege können in einer regelmäßigen Anordnung auf der Innenseite angeordnet sein, beispielsweise in verschiedenen Mustern, um eine Beeinflussung der Wärmeregulation und des Abtransportes von Feuchtigkeit zu ermöglichen. Insbesondere kann eine lineare Anordnung der Noppen und/oder Stege in Richtung von dem distalen Ende zum proximalen Ende erfolgen, um die natürliche Strömungsrichtung erwärmter Luft nach oben auszunutzen und schnellstmöglich diese Luft mit der darin enthaltenen Feuchtigkeit abzuleiten. Ebenfalls ist es möglich, die Noppen und/oder Stege so anzuordnen, dass sie in Umfangsrichtung verlaufende Kanäle, gegebenenfalls spiralförmige Kanäle, aufweisen, um eine Leitungsfunktion der Luftströmung zu erreichen. Die Noppen können in Umfangsrichtung versetzt zueinander angeordnet sein, um bestimmte Vorzugsrichtungen von Strömungen erreichen zu können.

Die Noppen und/oder Stege können auf der gesamten Innenseite angeordnet sein, um eine möglichst gleichmäßige Anlagen und Druckverteilung auf der Stumpfoberfläche zu erreichen. Neben der Anordnung vollflächiger Muster oder der kompletten Bestückung der Innenseite mit gleichmäßig verteilten Noppen und/oder Stege sind nur partielle Bestückungen mit Noppen und/oder Stegen möglich und vorgesehen, so dass nur einzelne Noppen- oder Stegbereiche auf der Innenseite angeordnet sind, um an besonders problematischen Stellen des Stumpfes eine natürliche Wärmeregulation ermöglichen zu können. Wichtig ist dabei, dass über die Steg- oder Noppenzwischenräume der Transport sowohl von erwärmter Luft als auch Feuchtigkeit gegeben ist.

Die Noppen und/oder Stege können eine zur Hautoberfläche gerichtete ebene Oberfläche aufweisen, wobei auch eine leichte Wölbung von der Hautoberfläche weg möglich ist, um bei einer Druckbelastung einklemmende Hautbereiche zu vermeiden. Die Oberfläche der Noppen und/oder Stege kann eine Rauhigkeit aufweisen, um einen verbesserten Komfort aufgrund der nicht vollständig glatten Oberfläche zu erreichen.

Das Verhältnis von Noppenlänge zu Noppenbreite kann zwischen 0,8 und 2,5 liegen, wodurch gewährleistet wird, dass für den Abtransport der Luft ausreichend große Kanäle oder Zwischenräume vorhanden sind und andererseits das Tragegefühl der Patienten weiterhin angenehm bleibt.

Das Verhältnis von Noppenlänge zu Noppenhöhe kann zwischen 1,5 und 2,5 liegen, wodurch gewährleistet wird, dass die Noppen nicht zu hoch sind und ein zu tiefes Eindrücken der Noppen in die Hautoberfläche verhindert wird. Andererseits ist die Noppenhöhe ausreichend groß, um einen Luftstrom zu gewährleisten, um Wärme und Feuchtigkeit abzutransportieren.

Der Abstand zwischen zwei Noppen kann zwischen der Hälfte der Noppenhöhe und dem 1,5fachen der Noppenhöhe liegen, so dass eine ausreichende Luftzirkulation vorhanden ist. Die Kanäle sind dann ausreichend groß, um den benötigten Abtransport der Wärme in Form von Konvektion und Schweißverdunstung zu ermöglichen.

Die Noppen und/oder Stege selbst können eine Höhe zwischen 0,25 mm und 2 mm, bevorzugt zwischen 0,5 mm und 1mm aufweisen, um einerseits eine ausreichende Höhe für die Luftzirkulation und den Schweißabtransport und andererseits eine ausreichende Stabilität gegen ein Umklappen oder ein Verschieben der Lineraußenseiten gegenüber der Hautoberfläche bereitzustellen. Die Steg- und/oder Noppenhöhe kann zwischen 5% und 25% der relativen Wandstärke des Liners betragen, also der Wanddicke des Liners an der Basis der Noppe oder des Steges.

Die Noppenform ist bevorzugt rund, insbesondere oval oder rechteckig, wodurch eine Ausbildung von Oberflächenkanälen und eine gerichtete Strömung erreicht werden können.

Das Verhältnis von Noppenhöhe zu Noppenbreite oder Steghöhe und Stegbreite ist bevorzugt kleiner als 1, so dass die Breite der Noppen oder Stege größer als deren Höhe ist, wodurch verhindert wird, dass die zwischen den Noppen und/oder Stegen befindlichen Kanäle bei einer großen Druckbelastung durch sich verformende Noppen und/oder Stegen blockiert werden. Das vorgeschlagene Verhältnis stellt sicher, dass die Zwischenräume im Wesentlichen durchgängig bleiben.

Zur Unterstützung der Wärmeregulierung können eine oder mehrere Anschlusseinrichtungen für eine Belüftungseinrichtung vorgesehen sein, so dass beispielsweise Pumpen oder Ventilatoren an den Liner angeschlossen werden können, um Luft abzusaugen, hineinzudrücken oder auch Feuchtigkeit aus dem Linerinnenraum abzutransportieren.

Die insbesondere durch die Noppen ausgebildeten Kanäle können bis zu dem proximalen oder distalen Rand des Prothesenliners reichen. Sie können als Belüftungs- oder Absaugkanäle ausgebildet sein, wodurch es möglich ist, dass eine Durchlüftung der Hautoberfläche, die nicht mit den Noppenoberflächen oder Stegoberflächen oder den Kanalzwischenmaterial in direktem Kontakt steht, stattfindet. Bei einer Luftzufuhr von außen ist eine stete Durchlüftung der Zwischenräume oder Kanäle gewährleistet. Die Ausgestaltung als Absaugkanäle sieht vor, dass diese in strömungstechnischer Verbindung zu einer Pumpeinrichtung stehen, beispielsweise einer Saugpumpe, die mechanisch oder elektrisch angetrieben wird. Ebenso ist es möglich, eine Belüftung durchzuführen, wenn die Kanäle mit einer Belüftungseinrichtung in Verbindung stehen, beispielsweise einer druckseitig angeschlossenen Pumpe. Durch eine Umschaltung der Pumpe von Saug- auf Druckbetrieb ist es möglich, die Kanäle und die Pumpe für unterschiedliche Betriebsarten zu nutzen, nämlich als aktive Belüftung oder aktive Absaugung. Reichen die Kanäle bis zu dem proximalen und/oder distalen Rand des Liners, findet eine Be- und Entlüftung der Kanäle und damit des Freiraumes oberhalb der Haut über einen Differentialdruck statt, der zwischen dem Inneren des Liners und der Umgebung herrscht. Bei einem Überdruck entweicht Luft aus dem Liner, bei einem Unterdruck strömt Luft in den Liner hinein, genauer gesagt in die Kanäle, die durch die Noppen ausgebildet werden. Sowohl bei der passiven, mit Differentialdruck betriebenen, als auch bei der aktiven, mit eine Pumpe bewirkten Belüftung oder Entlüftung kann der proximale Rand des Liners geschlossen ausgebildet sein, wenn eine Zufuhr von Luft über Be- oder Entlüftungsöffnungen, ggf. mit einem Ventil versehen, erfolgt. Der proximale Rand des Liners kann durch den Liners selbst oder mit einer Manschette abgedichtet sein, so dass der Schweißdampf im distalen Endbereich abgeführt wird, beispielsweise durch eine entsprechende Auslassöffnung, ggf. mit einem Ventil. Der Volumenausgleich bei einem abgedichteten Rand findet durch die Schweißproduktion und den Phasenwechsel von flüssig zu gasförmig bei der Verdunstung statt. Wird nicht die gesamte Feuchtigkeit verdunstet, kann der Schweiß in flüssiger Form abgeführt werden, was zu einer trockenen Hautoberfläche und einer dadurch verbesserten Hygiene führt. Soll Unterdruck an den Liner angelegt werden, kann dies neben einer separaten Pumpe auch über einen ggf. zwischen dem Schaft und dem Liner anliegenden Unterdruck geschehen, indem eine strömungstechnische Verbindung von dem Schaftinnenraum zu dem Linerinnenraum hergestellt wird. So kann auf eine gesonderte Pumpe verzichtet werden.

In dem Liner kann zumindest ein Anschluss zum Be- oder Entlüften angeordnet sein, der mit den Kanälen in strömungstechnischer Verbindung steht, um zu gewährleisten, dass Wärme und Feuchtigkeit aus dem Liner abtransportiert werden oder eine ausreichende Belüftung der Hautoberfläche erfolgt. Der Anschluss kann an dem distalen Ende des Liners oder an dem proximalen Rand angeordnet sein. Die Anordnung des Anschlusses an dem distalen Ende hat den Vorteil, dass er in der Regel an dem während des Stehens und Gehens tiefsten Punkt des Liners liegt, wodurch sich schwerkraftbedingt eine Fließrichtung für Feuchtigkeit ergibt, die zum Abtransport der Feuchtigkeit genutzt werden kann. Der Anschluss kann mit einer Verriegelungseinrichtung für den Liner in einem Schaft gekoppelt sein und in einem sogenannten Shuttle-Lock integriert sein.

Eine Weiterbildung der Erfindung sieht vor, dass ein Phasenwechselmaterial an dem Liner angeordnet oder in dem Liner integriert ist. Mit der Verwendung eines Phasenwechselmaterials, auch PCM genannt, ist es möglich, einen kühlenden Effekt auf den Luftstrom auszuüben, der sich in dem Bereich der Kanäle oder des Zwischenraumes zwischen der Haut und dem Liner ergibt. Über die PCM ist es möglich, eine aktive Kühlung für den Nutzer des Liners bereitzustellen, indem die Enthalpie thermodynamischer Zustandsänderungen eines Speichermediums ausgenutzt wird. Zwischen der Hautoberfläche und dem Liner findet eine Belüftung in den Kanälen statt, so dass die Schweißverdunstung zur Kühlung beiträgt. Über das Phasenwechselmaterial oder die Phasenwechselmaterialien ist es möglich, eine verstärkte Kühlwirkung zu erzielen, beispielsweise indem der Luftstrom innerhalb des Liners gleichzeitig von dem Phasenwechselmaterial gekühlt wird.

Es ist möglich, dass die Kanäle in einem direkten Kontakt mit dem Phasenwechselmaterial stehen, beispielsweise indem zumindest ein Teil der Kanalwände aus einem Phasenwechselmaterial besteht oder mit diesem beschichtet ist, so dass die Kanäle in direkter strömungstechnischer Verbindung mit einem Phasenwechselmaterial stehen. Zusätzlich ist es möglich, dass eine Pumpe vorgesehen ist, um die Luftströmung zu befördern. Die Durchströmung der Kanäle kann durch einen angelegten Unterdruck erfolgen, wodurch die Verdunstung des Körperschweißes erleichtert wird, was zur verbesserten Kühlung beiträgt. Sofern keine oder eine verringerte Kühlung gewünscht wird, kann über eine Schaltung oder Steuerung vorgesehen werden, dass der Luftaustausch reduziert wird, so dass eine Verringerung oder Vermeidung eines Wärmetransportes bewirkt wird.

Nachfolgend werden Ausführungsbeispiele der Erfindung anhand der beigefügten Figuren näher erläutert. Es zeigen:
- Figur 1 -: einen Radialschnitt durch einen Liner und eine Detaildarstellung;
- Figur 2-: einen Radialschnitt in Draufsicht gemäß Figur 1;
- Figur 3 -: einen Radialschnitt durch einen einstückigen Liner mit Detaildarstellung;
- Figur 4 -: einen Radialschnitt in Draufsicht und eine Detaildarstellung mit separaten Noppen, die nicht von der Erfindung umfasst sind;
- Figur 5 -: eine Querschnittsdarstellung mit einer Detailansicht;
- Figur 6 -: eine Querschnittsdarstellung gemäß Figur 5 in Draufsicht;
- Figur 7 -: eine einstückige Variante eines Liners gemäß Figur 5;
- Figur 8 -: eine Draufsicht auf einen Querschnitt eines Liners mit separaten Noppen, die nicht von der Erfindung umfasst sind;
- Figur 9 -: eine Draufsicht auf zwei Ausführungsformen;
- Figur 10 -: eine Variante der Figur 9;
- Figur 11 -: einen Radialschnitt;
- Figur 12 -: eine Detaildarstellung der Figur 11;
- Figur 13 -: eine Querschnittsansicht der Figur 11;
- Figur 14 -: eine Detailansicht der Figur 13;
- Figur 15 -: eine Draufsicht auf eine Variante; sowie
- Figur 16 -: eine Draufsicht auf eine weitere Variante.

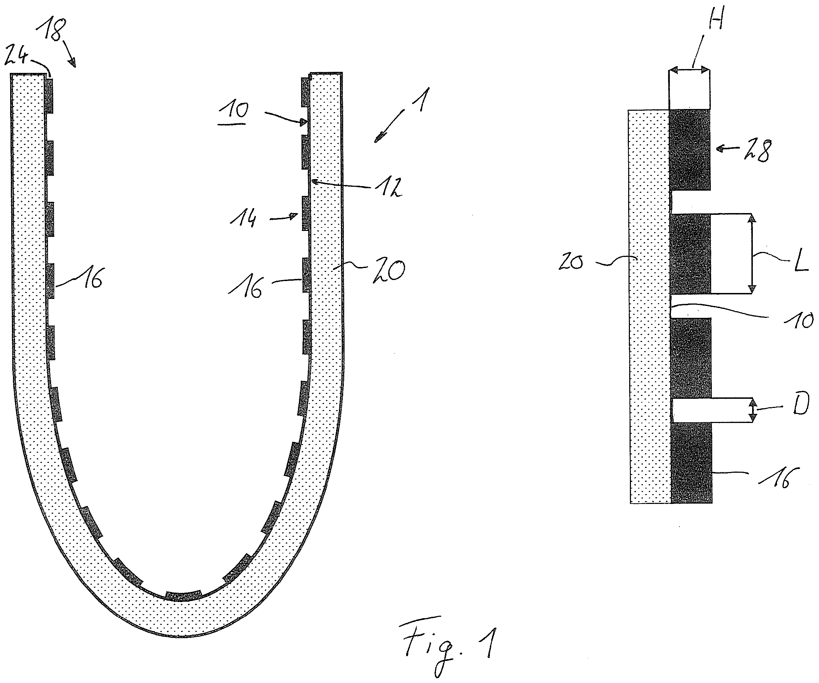

Figur 1 zeigt in der linken Darstellung einen Liner 1 mit einem Radialschnitt entlang der Längserstreckung, in der nur die Schnittebene dargestellt ist. Der Liner 1 ist als geschlossener Liner ausgebildet und weist ein geschlossenes, distales Ende und eine Einstiegsöffnung 18 auf, über die ein nicht dargestellter Stumpf einer Gliedmaße in den Liner 1 eingeführt werden kann. Der Liner 1 weist einen Grundkörper 10 aus einem flexiblen, wasserundurchlässigen und vorteilhafterweise elastischen Material auf. Der Grundkörper 10 weist eine im angelegten Zustand dem Stumpf zugewandte Innenseite 14 und eine der Hautoberfläche abgewandte Außenseite 12 auf. Die Außenseite 12 ist geschlossen ausgebildet, so dass keine Feuchtigkeit von dem Stumpf durch den Grundkörper 10 nach außen dringen oder durch den Grundkörper 10 an den Stumpf gelangen kann. Als Material für den Grundkörper 10 kann Silikon oder ein Kunststoff, beispielsweise Polyurethan verwendet werden. Auf der Innenseite 14 des Grundkörpers 10 sind eine Vielzahl gleichartiger Noppen 16 ausgebildet, die in Richtung auf die Hautoberfläche über den Grundkörper 10 hinausragen. Auf der Außenseite 12 des Grundkörpers 10 ist eine Beschichtung 20 aufgebracht, die aus einem Material besteht, das von dem Material des Grundkörpers 10 verschieden ist. Durch die Noppen 16, die eine zur Hautoberfläche hingewandte, ebene Oberfläche 28 aufweisen, wird eine Belüftung der Hautoberfläche an denjenigen Bereichen ermöglicht, an denen die Noppen 16 nicht auf der Hautoberfläche aufliegen. Dadurch ist es möglich, Feuchtigkeit und Wärme aus dem Liner 1 entlang der Hautoberfläche abzutransportieren, so dass warme Luft und Feuchtigkeit am distalen Rand 24 des Liners austreten kann.

In der rechten Darstellung der Figur 1 ist eine vergrößerte Detailansicht einer Linerwand mit einer außenseitigen Beschichtung 20, dem Grundkörper 10 sowie den davon hervorstehenden, zur Innenseite gerichteten Noppen 16 zu erkennen. Die Noppen 16 weisen eine ebene Oberfläche 28 und eine Noppenhöhe H auf, die kleiner als die Noppenlänge L ist. Das Verhältnis von Noppenlänge L zu Noppenhöhe H liegt zwischen 1,5 und 2,5, wobei es sich als vorteilhafter herausgestellt hat, wenn die Noppenlänge L in einem Bereich zwischen 0,7mm und 1,2mm ausgebildet ist. Die Abstände D zwischen zwei Noppen 16 sind vorteilhafterweise geringer als die Noppenhöhe H und betragen im dargestellten Ausführungsbeispiel ungefähr die Hälfte der Noppenhöhe. Es kann auch sein, dass die Abstände größer gewählt sind, so dass der Abstand D zwischen zwei Noppen 16 bis zum 1,5fachen der Noppenhöhe H betragen kann. Der Abstand kann zwischen 0,2mm und 1,2mm betragen.

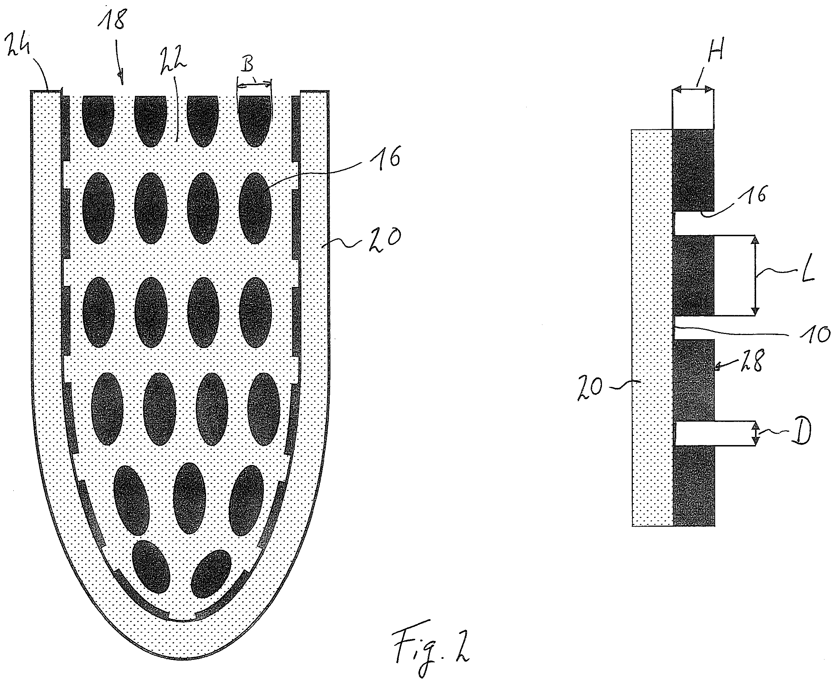

In der Figur 2 ist eine alternative Darstellung des Liners 1 gemäß Figur 1 gezeigt. In der linken Darstellung ist zu erkennen, dass die Form der Noppen 16 oval ist, der Abstand D ist der jeweils kleinste Abstand zwischen zwei Noppen 16, die Noppenlänge L bezeichnet dabei die größere Längserstreckung, die Noppenbreite B die kleinere Ausdehnung der Noppen 16. Das Verhältnis von Noppenlänge zu Noppenbreite kann zwischen 1 und 2,5 liegen. Die Anordnung der Noppen 16 verläuft im Wesentlichen gleichmäßig über die gesamte Innenseite 14 des Grundkörpers 10, wobei sich zwischen den Noppen 16 nach innen offene, unterbrochene Kanäle 22 ausbilden, die einmal im Wesentlichen von distal nach proximal verlaufen und gleichzeitig aufgrund der Anordnung der Noppen 16 auf gleicher Höhe umlaufende Kanäle ausbilden, die sich mit den Kanälen 22 in proximal-distal-Richtung kreuzen, so dass ein freier Durchgang von Wärme und Feuchtigkeit von allen nicht von Noppenoberflächen 28 abgedeckten Körperpartien zum proximalen Rand 22 möglich ist. Der Austritt von Wärme und Feuchtigkeit erfolgt dabei in dem Zwischenraum zwischen dem Grundkörper 10 und der Hautoberfläche.

In der rechten Darstellung ist zu erkennen, dass die Beschichtung 22 aus einem von dem Material des Grundkörpers 10 verschiedenen Material besteht, der Grundkörper 10 und die Noppen 16 sind einteilig bzw. einstückig ausgebildet und weisen eine gute Haftfähigkeit bei Anlage auf der Haut auf. Die außenseitige Beschichtung 20 kann aus einem Material gewählt sein, das besonders gute mechanische Eigenschaften und eine gute Dauerhaltbarkeit aufweist, was nicht unbedingt mit einer Hautverträglichkeit zusammenfallen muss, so dass für die Gestaltung der Liner 1 durch die Verwendbarkeit zweier verschiedener Materialien große Freiheiten vorhanden sind.

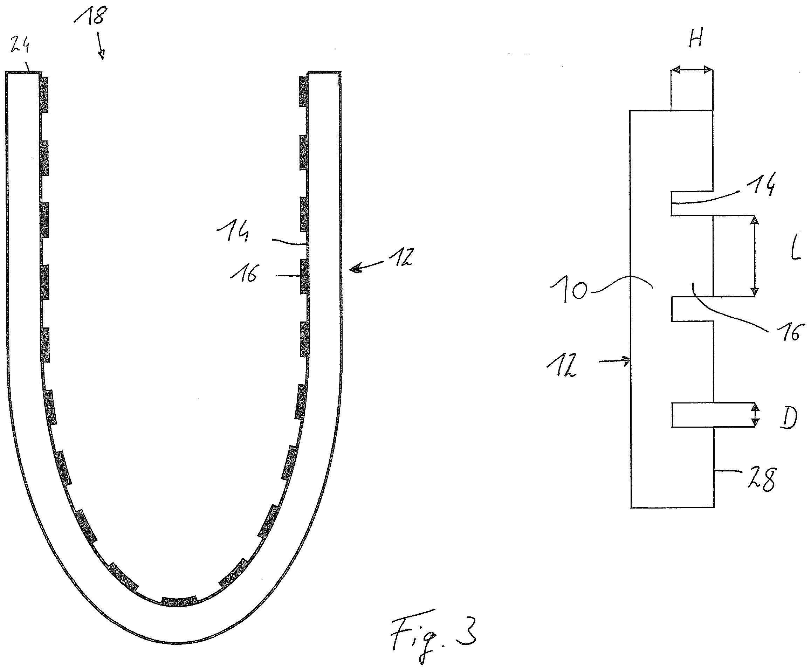

Figur 3 zeigt einen Radialschnitt analog zur Figur 1, in der rechten Darstellung wird deutlich, dass der gesamte Liner 1 aus einem einzigen Material hergestellt ist und die Noppen 16 auf der Innenseite 14 des Grundkörpers 10 einstückig herausgeformt sind.

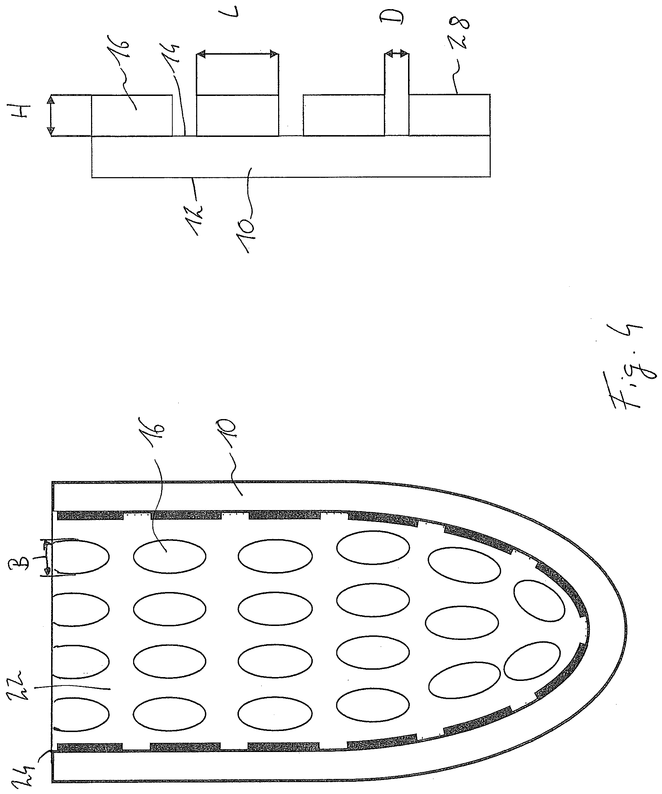

Eine Variante der Figur 3 ist in der Figur 4 dargestellt, bei der die Noppenform der gemäß Figur 2 entspricht, die Noppen 16 und der Grundkörper 10 jedoch nicht einstückig ausgebildet sind, sondern nachträglich auf dem Grundkörper 10 angebracht wurden, beispielsweise durch Aufkleben oder durch nachträgliches Vernetzen und stoffschlüssiges Verbinden. Der Liner 1 gemäß Figur 4 sieht die Verwendung von nur einem Material vor. Durch das nachträgliche Aufbringen der Noppen 16 kann eine größere Variationsbreite bei der Anfertigung von Linern abgedeckt werden, da auf individuelle Besonderheiten Rücksicht genommen werden kann.

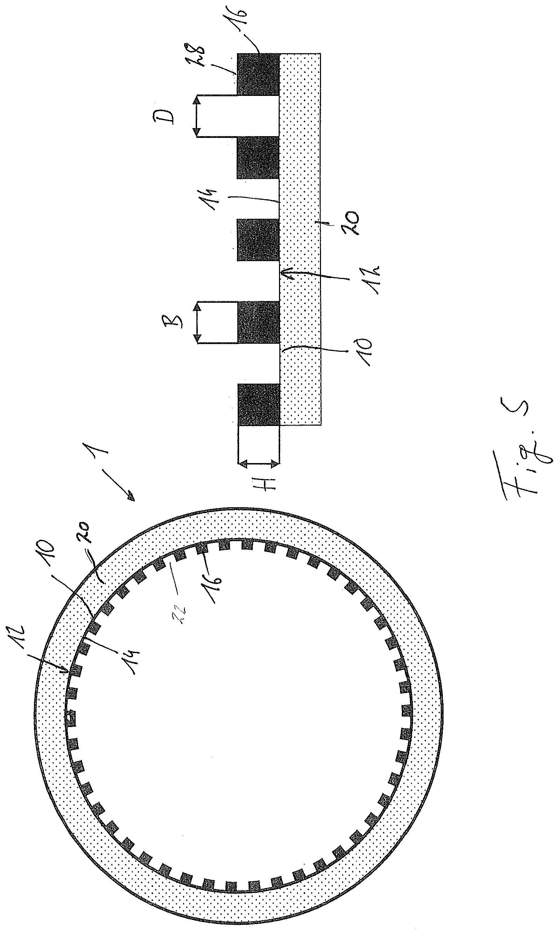

In der Figur 5 ist ein Querschnitt durch einen Liner 1 gemäß Figur 1 senkrecht zur Längserstreckung gezeigt. Durch die gleichmäßige Anordnung der Noppen 16 auf der Innenseite 14 des Grundkörpers 10 ergeben sich die Kanäle 22. Die Figur 5 zeigt den geschlossenen Querschnitt des Liners 1, so dass der Liner 1 den nicht dargestellten Stumpf vollständig umschließen kann. In dem Ausführungsbeispiel gemäß Figur 5 ist die Noppenhöhe H gleich der Noppenbreite B gleich dem Abstand D zwischen zwei Noppen 16 und damit auch der Breite der Kanäle 22.

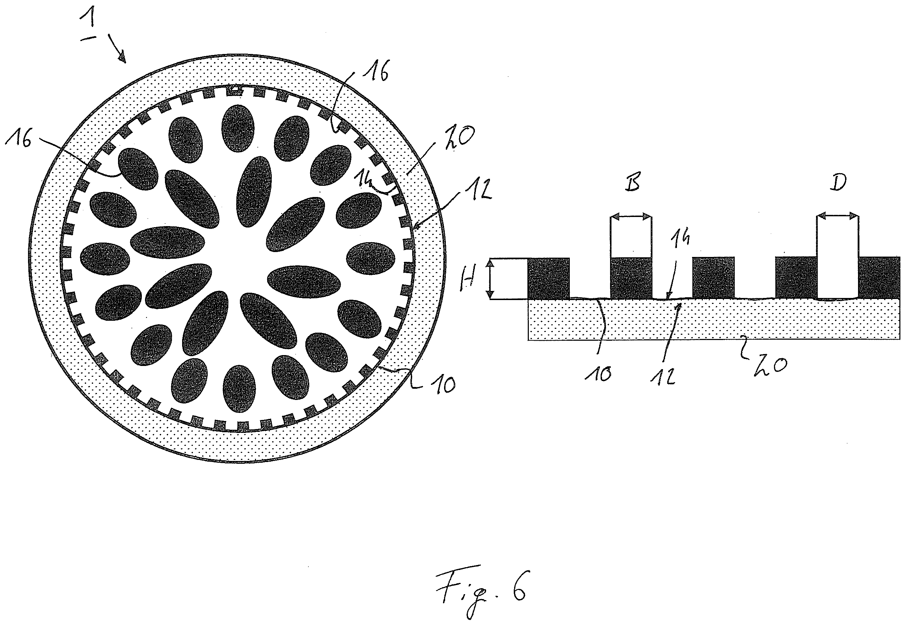

Figur 6 zeigt eine alternative Darstellung des Liners 1 gemäß Figur 6, in der die Anordnung der Noppen 16 besser zu erkennen ist. Die Noppen 16 erstrecken sich entlang der im Wesentlichen zylindrischen Seitenwand des Grundkörpers 10 ebenso wie in dem konisch zulaufenden distalen Endbereich, wobei im tiefsten Punkt des Liners keine Noppen 16 vorgesehen sind.

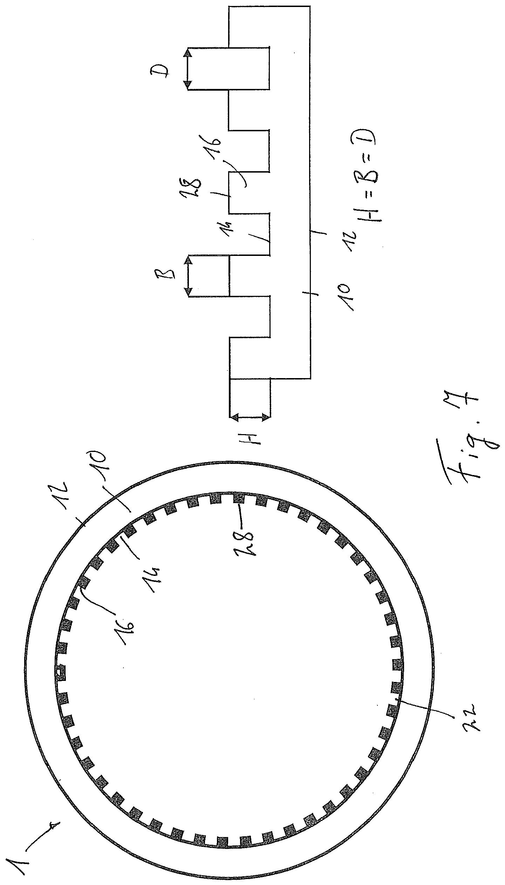

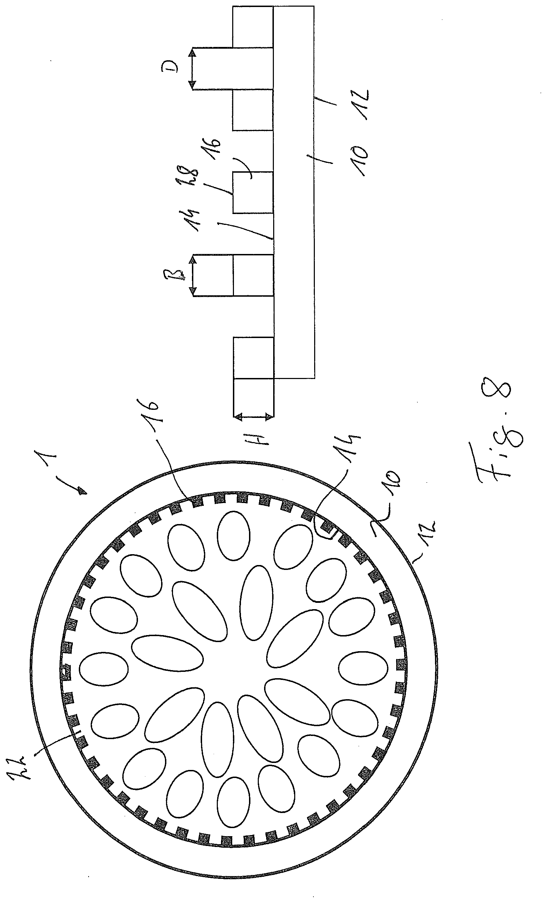

Figur 7 zeigt eine Querschnittsdarstellung eines Liners gemäß Figur 3, Figur 8 einen Querschnitt durch einen Liner gemäß Figur 4. Der Unterschied der Ausführungsformen gemäß Figuren 7 und 8 besteht darin, dass der Grundkörper 10 keine Beschichtung auf der Außenseite 12 aufweist. Das Material der Noppen 16 im Ausführungsbeispiel der Figuren 4 und 8 kann auch von dem Material des Grundkörpers 10 abweichen.

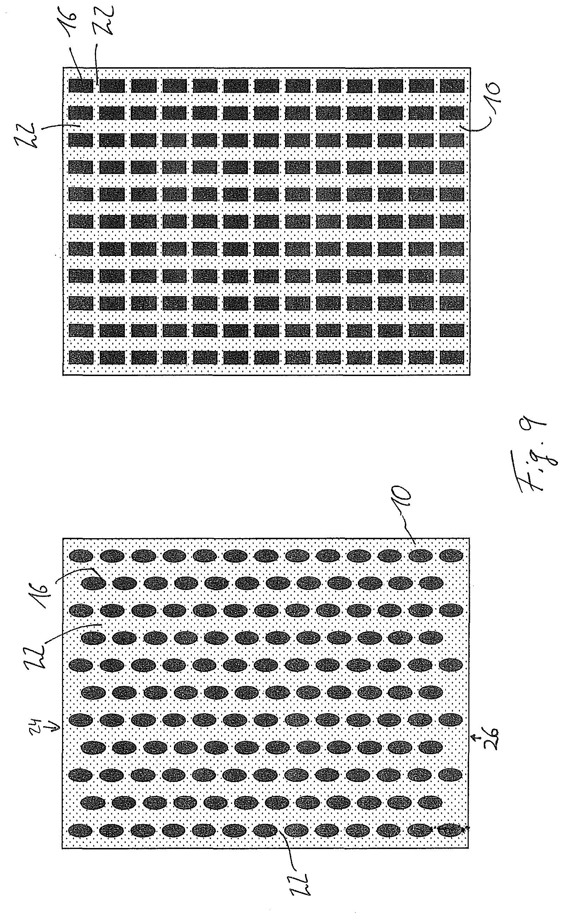

In der Figur 9 ist eine Draufsicht auf die Innenseite 14 eines Grundkörpers 10 mit unterschiedlichen Noppenformen dargestellt. Die linke Darstellung sieht ovale Noppen 16 vor, die rechte Darstellung rechteckige Noppen 16. In Längserstreckung vom distalen Rand 26 zum proximalen Rand 24 verlaufen die Noppen 16 im Wesentlichen parallel, in der linken Darstellung sind die Noppen höhenversetzt, so dass sich diagonal verlaufende Kanäle 22 neben den parallel verlaufenden Kanälen 22 ausbilden, in der rechten Darstellung sind die Noppen 16 auf dem Grundkörper 10 gleichmäßig verteilt und laufen jeweils parallel zueinander. Der Grundkörper 10 kann mit nicht dargestellten Verschluss- oder Befestigungselementen versehen sein, so dass er um einen Stumpf oder eine Gliedmaße herumgewickelt und daran festgelegt sein kann, so dass ein am distalen Rand 26 und am proximalen Rand 24 offener Liner entsteht, der als Interface für eine prothetische oder orthetische Versorgung dienen kann. Grundsätzlich ist es auch möglich, einen hülsenförmigen oder schlauchförmigen Liner mit einem offenen distalen Ende vorzusehen.

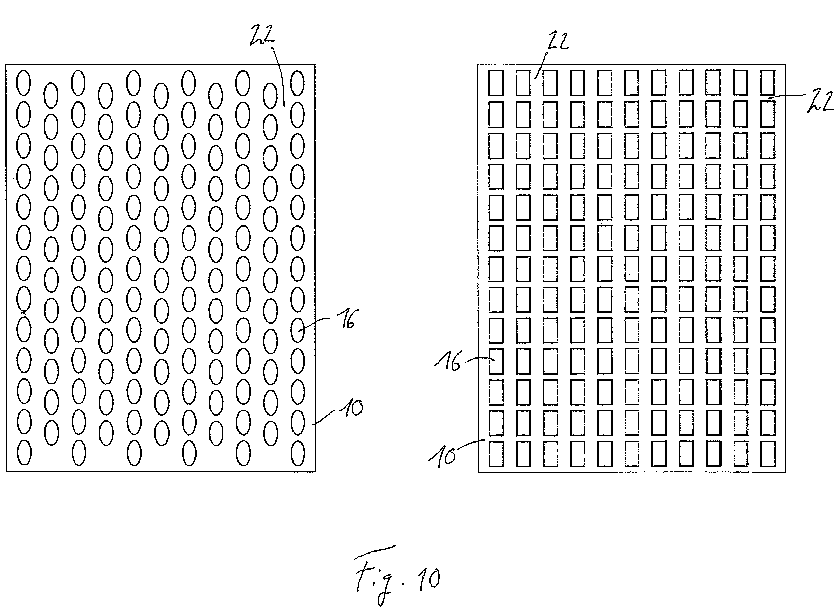

Die Noppen im Ausführungsbeispiel gemäß Figur 9 können aus einem vom Grundkörper 10 verschiedenen Material ausgebildet sein. In der Figur 10 ist die Draufsicht auf die Grundkörper gemäß Figur 9 mit Noppen 16 aus dem gleichen Material wie dem Grundkörper 10 dargestellt. Die Noppen 16 gemäß Figur 10 können beim Urformprozess des Grundkörpers 10 mit angeformt oder nachträglich aufgebracht werden. Die Noppen 16 auf beiden Grundkörpern 10 bilden eine strukturierte Oberfläche mit abwechselnden Erhöhungen und Vertiefungen, so dass in den Noppenzwischenräumen eine Luftzirkulation und ein Abtransport von Feuchtigkeit erfolgen kann.

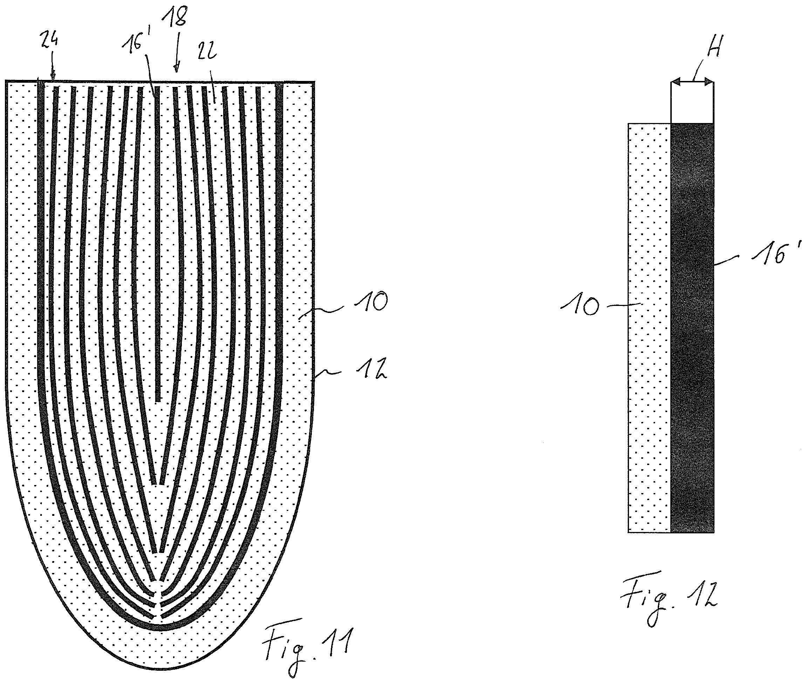

In der Figur 11 ist der Liner 1 in einem Radialschnitt mit einem Grundkörper 10 und einer Außenseite 12 dargestellt. Die Einführöffnung 18 am distalen Rand 24 ermöglicht ein Umschlagen und Einsteigen in den Liner 1. Auf der Innenseite des Grundkörpers 12 sind Stege 16' vorgesehen, die sich radial nach innen erstrecken, so dass zwischen den Stegen 16' Kanäle 22 ausgebildet sind, die sich von dem geschlossenen distalen Ende bis zum offenen proximalen Rand 24 erstrecken. Die Kanäle 22 sind im Wesentlichen gradlinig an der Innenseite des Grundkörpers 10 orientiert und ermöglichen einen direkten Abtransport von Schweiß sowie einen Luftaustausch. Der Grundkörper 10 ist geschlossenwandig und im Wesentlichen wasserundurchlässig ausgebildet.

Figur 12 zeigt eine Detaildarstellung des Liners 1 mit dem Grundkörper 10 und dem radial nach innen ragenden Steg 16'. Die Höhe H des Steges 16' ist so bemessen, dass einerseits eine ausreichend große Stabilität gegenüber einer Verformung bei Aufbringen einer Druckkraft vorhanden ist und andererseits ein ausreichender Abstand zwischen der Innenseite des Grundkörpers 10 und der nicht dargestellten Hautoberfläche bereitgestellt wird, um den Abtransport von Luft und Feuchtigkeit zu ermöglichen.

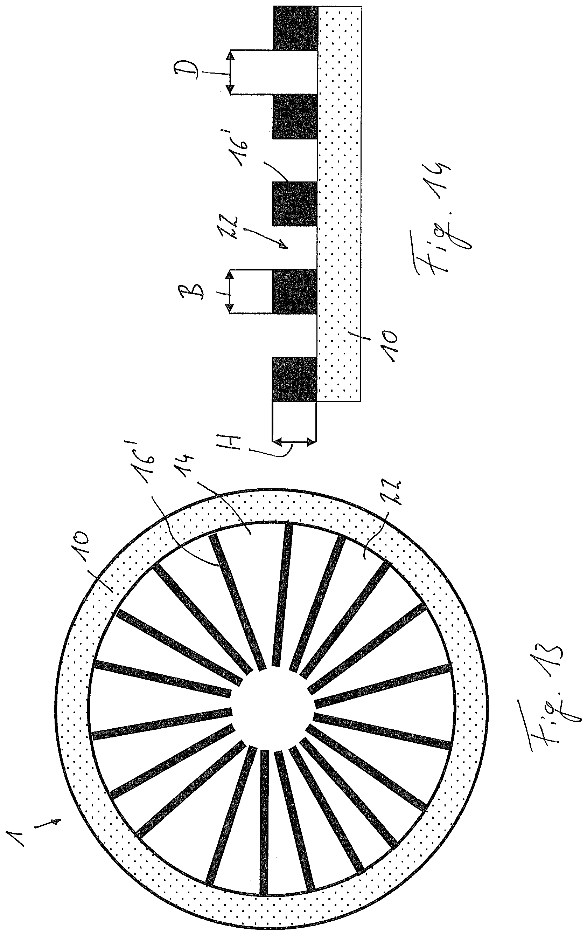

Figur 13 zeigt einen Querschnitt durch den Liner 1, aus der Figur ist zu erkennen, dass der Liner 1 einen im Wesentlichen kreisförmigen Querschnitt und eine geschlossene Struktur des Grundkörpers 10 aufweist. Die als Stege 16' ausgebildeten Vorsprünge, die von der Innenseite 14 des Grundkörpers 10 radial nach innen ragen, laufen vom proximalen Rand bis zur distalen Spitze. Zwischen den Stegen 16' sind die Kanäle 22 ausgebildet, um Luft und Feuchtigkeit abzutransportieren. Eine Schnittdarstellung ist in der Figur 14 gezeigt, aus der zu erkennen ist, dass die Höhe H und die Breite B der Stege 16' einander im Wesentlichen entsprechen, so dass sich ein quadratischer Querschnitt, ausgehend von dem Grundkörper 10, für den Steg 16' ergibt. Der Abstand D zwischen zwei Stegen 16' entspricht im Wesentlichen der Breite B des Steges 16', ein möglicher Abmessungsbereich für die Höhe H, die Breite B und der Abstand D liegt zwischen 0,3 und 0,7 mm, ist darauf jedoch nicht beschränkt.

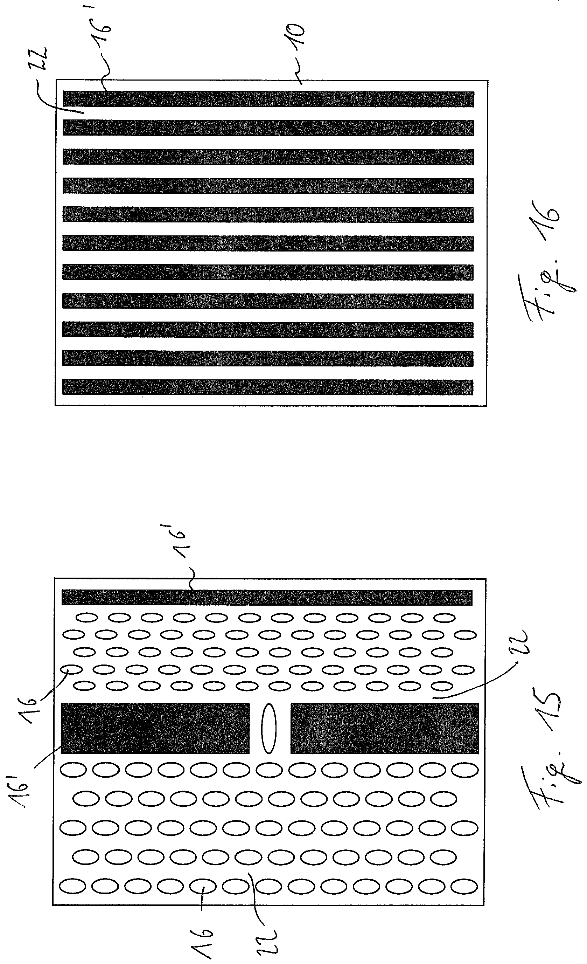

Figur 15 zeigt eine erste Variante der Innenseite eines Liners in einer Draufsicht, bei der neben Noppen 16 in unterschiedlichen Größen und Orientierungen in ovaler Form Stege 16' auf dem Grundkörper 10 aufgebracht oder ausgebildet sind. Zwischen den Stegen 16' und Noppen 16 sind die Kanäle 22 ausgebildet. Der Figur 15 ist zu entnehmen, dass die Stege 16' nicht durchgängig vom distalen bis zum proximalen Ende verlaufen müssen, vielmehr sind auch Unterbrechungen möglich. Im dargestellten Ausführungsbeispiel ist zwischen zwei Stegabschnitten 16' eine Noppe 16 angeordnet. Grundsätzlich ist es möglich, Noppen 16 und Stege 16' unterschiedlicher Größen und Orientierungen auf der Innenseite des Grundkörpers 10 anzuordnen.

Eine weitere Variante ist in der Figur 16 dargestellt, bei der eine Vielzahl von Stegen 16' gleichmäßig zueinander beabstandet auf der Innenseite des Grundkörpers 10 angeordnet sind. Zwischen den Stegen 16' sind dementsprechend gleichmäßig ausgebildete, gradlinig verlaufende Kanäle 22 ausgebildet.

## Patentansprüche

1. Liner zur Anlage an einer Hautoberfläche mit einem Grundkörper (10) aus einem flexiblen, wasserundurchlässigen Material mit einer im angelegten Zustand von der Hautoberfläche abgewandten Außenseite (12) und einer im angelegten Zustand der Hautoberfläche zugewandten Innenseite (14), wobei der Grundkörper (10) eine geschlossene Außenseite (12) aufweist und auf der Innenseite (14) zur Hautoberfläche hin offene Kanäle (22) angeordnet sind, entlang derer Feuchtigkeit und Wärme abgeführt werden, wobei die Kanäle (22) durch Noppen (16) und/oder Stege (16') gebildet und die Noppen (16) und/oder Stege (16') einstückig mit dem Grundkörper ausgebildet sind.

2. Liner nach Anspruch 1, wobei der Grundkörper (10) einen geschlossen Querschnitt mit einer proximalen Einstiegsöffnung (18) aufweist.

3. Liner nach einem der voranstehenden Ansprüche, wobei auf der Außenseite (12) eine Beschichtung (20) aus einem von dem Material des Grundkörpers (10) abweichenden Material aufgebracht ist.

4. Liner nach einem der voranstehenden Ansprüche, wobei die Noppen (16) und/oder Stege (16') in einer regelmäßigen Anordnung auf der Innenseite (14) angeordnet sind.

5. Liner nach einem der voranstehenden Ansprüche, wobei die Noppen (16) und/oder Stege (16') auf der gesamten Innenseite (14) angeordnet sind.

6. Liner nach einem der voranstehenden Ansprüche, wobei die Noppen (16) und/oder Stege (16') eine zur Hautoberfläche gerichtete ebene Oberfläche (28) aufweisen.

7. Liner nach einem der voranstehenden Ansprüche, wobei das Verhältnis von Noppenlänge (L) zu Noppenbreite (B) zwischen 0,8 und 2,5 liegt und/oder dass das Verhältnis von Noppenlänge (L) zu Noppenhöhe (H) zwischen 1,5 und 2,5 liegt.

8. Liner nach einem der voranstehenden Ansprüche, wobei der Abstand (D) zwischen zwei Noppen (16) und/oder Stegen (16') zwischen der Hälfte der Noppen- oder Steghöhe (H) und dem 1,5fachen der Noppen- oder Steghöhe (H) liegt.

9. Liner nach einem der voranstehenden Ansprüche, wobei das Verhältnis von Noppen- oder Steghöhe (H) zur Noppen- oder Stegbreite (B) kleiner 1 ist.

10. Liner nach einem der voranstehenden Ansprüche, wobei die Kanäle (22) bis zu einem proximalen oder distalen Rand (24, 26) reichen und/oder als Absaug- oder Belüftungskanäle ausgebildet sind.

11. Liner nach einem der voranstehenden Ansprüche, wobei zumindest eine Anschlusseinrichtung für eine Belüftungseinrichtung vorgesehen ist.

12. Liner nach einem der voranstehenden Ansprüche, wobei in dem Liner ein Anschluss zum Be- oder Entlüften angeordnet ist, der mit den Kanälen (22) in strömungstechnischer Verbindung steht.

13. Liner nach einem der voranstehenden Ansprüche, wobei ein Phasenwechselmaterial an dem Liner angeordnet oder in dem Liner integriert ist.

14. Liner nach Anspruch 13, wobei die Kanäle (22) mit einem Phasenwechselmaterial in strömungstechnischer Verbindung stehen.

## Claims

1. A liner for application to a surface of the skin, having a basic body (10) produced from a flexible, watertight material and having an outside surface (12) which, in the applied state, is remote from the surface of the skin and an inside surface (14) which, in the applied state, faces the surface of the skin, wherein the base body (10) has a closed outer side (12) and channels (22) which are open toward the surface of the skin are arranged on the inside surface (14), along which moisture and heat are dissipated, wherein the channels (22) are formed by nubs (16) and/or webs (16') and the nubs (16) and/or webs (16') are formed integrally with the base body.

2. The liner as claimed in claim 1, wherein the basic body (10) comprises a closed cross section with a proximal access opening (18).

3. The liner as claimed in one of the preceding claims, wherein a coating (20) which is produced from a material that deviates from the material of the basic body (10) is applied on the outside surface (12).

4. The liner as claimed in one of the preceding claims, wherein the nubs (16) and/or webs (16') are arranged in a regular arrangement on the inside surface (14).

5. The liner as claimed in one of the preceding claims, wherein the nubs (16) and/or webs (16') are arranged over the entire inside surface (14).

6. The liner as claimed in one of the preceding claims, wherein the nubs (16) and/or webs (16') comprise an even surface (28) which is directed to the surface of the skin.

7. The liner as claimed in one of the preceding claims, wherein the ratio of nub length (L) to nub width (B) is between 0.8 and 2.5 and/or that the ratio of nub length (L) to nub height (H) is between 1.5 and 2.5.

8. The liner as claimed in one of the preceding claims, wherein the distance (D) between two nubs (16) and/or webs (16') is between half of the nub or web height (H) and 1.5 times the nub or web height (H).

9. The liner as claimed in one of the preceding claims, wherein the ratio of nub or web height (H) to nub or web width (B) is less than 1.

10. The liner as claimed in one of the preceding claims, wherein the channels (22) reach up to a proximal or distal edge (24, 26) and/or are realized as suction or ventilation channels.

11. The liner as claimed in one of the preceding claims, wherein at least one connection device is provided for a ventilation device.

12. The liner as claimed in one of the preceding claims, wherein a connection for ventilation or aeration, which is fluidically connected to the channels (22), is arranged in the liner.

13. The liner as claimed in one of the preceding claims, wherein a phase change material is arranged on the liner or is integrated in the liner.

14. The liner as claimed in claim 13, wherein the channels (22) are fluidically connected to a phase change material.

## Revendications

1. Doublure pour l'application sur une surface épidermique, comportant un corps de base (10) en un matériau flexible, imperméable à l'eau, ayant une face extérieure (12) détournée de la surface épidermique à l'état appliqué et une face intérieure (14) tournée vers la surface épidermique à l'état appliqué,
dans laquelle
le corps de base (10) présente une face extérieure fermée (12), et des canaux (22) ouverts vers la surface épidermique sont disposés sur la face intérieure (14), le long desquels sont évacuées l'humidité et la chaleur, les canaux (22) étant formés par des bosses (16) et/ou par des barrettes (16'), et les bosses (16) et/ou les barrettes (16') étant réalisées d'un seul tenant avec le corps de base.

2. Doublure selon la revendication 1,
dans laquelle le corps de base (10) présente une section transversale fermée ayant une ouverture d'accès proximale (18).

3. Doublure selon l'une des revendications précédentes,
dans laquelle un revêtement (20) en un matériau différent du matériau du corps de base (10) est appliqué sur la face extérieure (12).

4. Doublure selon l'une des revendications précédentes,
dans laquelle en ce que les bosses (16) et/ou les barrettes (16') sont disposées en une disposition régulière sur la face intérieure (14).

5. Doublure selon l'une des revendications précédentes,
dans laquelle les bosses (16) et/ou les barrettes (16') sont disposées sur toute la face intérieure (14).

6. Doublure selon l'une des revendications précédentes,
dans laquelle les bosses (16) et/ou les barrettes (16') ont une surface plane (28) dirigée vers la surface épidermique.

7. Doublure selon l'une des revendications précédentes,
dans laquelle le rapport de la longueur (L) sur la largeur (B) de la bosse est compris entre 0,8 et 2,5, et/ou le rapport de la longueur (L) sur la hauteur (H) de la bosse est compris entre 1,5 et 2,5.

8. Doublure selon l'une des revendications précédentes,
dans laquelle la distance (D) entre deux bosses (16) et/ou barrettes (16') est comprise entre la moitié de la hauteur (H) de la bosse ou de la barrette et 1,5 fois la hauteur (H) de la bosse ou de la barrette.

9. Doublure selon l'une des revendications précédentes,
dans laquelle le rapport de la hauteur (H) de la bosse ou de la barrette sur la largeur (B) de la bosse ou de la barrette est inférieur à 1.

10. Doublure selon l'une des revendications précédentes,
dans laquelle les canaux (22) s'étendent jusqu'à un bord proximal ou distal (24, 26) et/ou sont réalisés sous forme de canaux d'aspiration ou de ventilation.

11. Doublure selon l'une des revendications précédentes,
dans laquelle il est prévu au moins un dispositif de raccordement pour un dispositif de ventilation.

12. Doublure selon l'une des revendications précédentes,
dans laquelle un raccord pour l'aération ou la ventilation est disposé dans la doublure, qui est en liaison fluidique avec les canaux (22).

13. Doublure selon l'une des revendications précédentes,
dans laquelle un matériau à changement de phase est disposé sur la doublure ou est intégré dans la doublure.

14. Doublure selon la revendication 13,
dans laquelle les canaux (22) sont en liaison fluidique avec un matériau à changement de phase.
